# EUROPEAN PATENT APPLICATION

(11) **EP 1 775 348 A1**
(43) Date of publication of application: **18.04.2007**
(21) Application number: 05022623.2
(22) Date of filing: 17.10.2005
(51) Int. Cl.: C12Q 1/68

(54) **Method of analyzing nucleic acid with mass spectrometry**

(71) Applicant: Max-Planck-Gesellschaft zur Förderung der Wissenschaften e.V., Berlin (DE)
(72) Inventor: Sauer, Sascha, 10711 Berlin (DE); Kepper, Pamela, 14167 Berlin (DE); Reinhardt, Richard, 14195 Berlin (DE); Lehrach, Hans, 14129 Berlin (DE)
(74) Representative: Vossius & Partner

(57) **Abstract**

This invention relates to a method of analyzing nucleic acid with regard to composition and/or sequence comprising (a) bringing a sample comprising said nucleic acid into contact with positively charged and/or charge-neutral groups, said groups being attached to a solid support and under conditions allowing binding of said nucleic acid to said groups; (b) performing one or more washing steps; (c) depositing MALDI matrix material onto said solid support which has been contacted with said nucleic acid in step (a); and (d) performing MALDI-MS of said nucleic acid.

### Annex to the application documents - subsequently filed sequences listing

SEQUENCE LISTING

<110>  Max-Planck-Gesellschaft zur Förderung der Wissenschaften
       e.V.

<120>  Method of analyzing nucleic acid with mass spectrometry

<130>  L 2105 EP

<140>  EP 05 02 2623.2
<141>  2005-10-17

<160>  13

<170>  PatentIn version 3.3

<210>  1
<211>  24
<212>  DNA
<213>  artificial sequence

<220>
<223>  primer

<400>  1
tatggcattt cacattcaca tgta                                              24


<210>  2
<211>  25
<212>  DNA
<213>  artificial sequence

<220>
<223>  primer

<400>  2
tatggcattt cacattcaca tgtag                                             25


<210>  3
<211>  26
<212>  DNA
<213>  artificial sequence

<220>
<223>  primer

<400>  3
tatggcattt cacattcaca tgtaat                                            26


<210>  4
<211>  19
<212>  DNA
<213>  artificial sequence

<220>
<223>  primer

<400>  4
aattgaatgg ctctaggac                                                    19

<210>  5
<211>  20
<212>  DNA
<213>  artificial sequence

<220>
<223>  primer

<400>  5
aattgaatgg ctctaggact                                                    20


<210>  6
<211>  21
<212>  DNA
<213>  artificial sequence

<220>
<223>  primer

<400>  6
aattgaatgg ctctaggact g                                                  21


<210>  7
<211>  23
<212>  DNA
<213>  artificial sequence

<220>
<223>  primer

<400>  7
aatgtgatat tttaaagggc cct                                                23


<210>  8
<211>  24
<212>  DNA
<213>  artificial sequence

<220>
<223>  primer

<400>  8
aagctctaaa acatggaaag gaaa                                               24


<210>  9
<211>  22
<212>  DNA
<213>  artificial sequence

<220>
<223>  primer

<400>  9
tcatgcaatg aaggggtctt at                                                 22


<210>  10
<211>  22
<212>  DNA
<213>  artificial sequence

<220>
<223>  primer

<400>  10
tgtgatgggt gctctagaca aa                                                22


<210>  11
<211>  22
<212>  DNA
<213>  artificial sequence

<220>
<223>  primer

<400>  11
gaaacctgca atacttgctg aa                                                22


<210>  12
<211>  19
<212>  DNA
<213>  artificial sequence

<220>
<223>  primer

<400>  12
aattgaatgg ctctaggac                                                    19


<210>  13
<211>  24
<212>  DNA
<213>  artificial sequence

<220>
<223>  primer

<400>  13
tatggcattt cacattcaca tgta                                              24

## Description

This invention relates to a method of analyzing nucleic acid with regard to composition and/or sequence comprising (a) bringing a sample comprising said nucleic acid into contact with positively charged and/or charge-neutral groups, said groups being attached to a solid support and under conditions allowing binding of said nucleic acid to said groups; (b) performing one or more washing steps; (c) depositing MALDI matrix material onto said solid support which has been contacted with said nucleic acid in step (a); and (d) performing MALDI-MS of said nucleic acid.

In this specification, a number of documents including patent applications and manufacturer's manuals is cited. The disclosure of these documents, while not considered relevant for the patentability of this invention, is herewith incorporated by reference in its entirety.

The detection of genome variation is presently one of the challenges in genetics^{1,2}. A number of applications comprise typing of single nucleotide polymorphisms (SNPs) to study complex traits. Moreover, SNPs can be useful in pharmacogenetics and in diagnostics^{3,4}. Matrix-assisted laser desorption/ionisation mass spectrometry (MALDI-MS)⁵ can be efficiently used for the detection of SNPs, mainly in diagnostic typing where it is likely that a low number of marker SNPs will be analyzed in large numbers of DNA samples⁶. Furthermore, MALDI-MS provides fast and accurate data accumulation that is based on the detection of an intrinsic physical property of a molecule, the mass.

Many procedures for the analysis of nucleic acids by MALDI-MS have been published during the last twelve years^{7,8}. A number of applications of these principles have been shown, comprising SNP genotyping and molecular haplotyping, epigenotyping, and quantitative and allele-specific expression analysis^{8,9}. The reaction sequence of these approaches is similar, starting with PCR amplification of a genomic region containing SNPs or methylation variation positions of interest, followed by removal of residual dNTPs and by primer extension to generate allele-specific products of SNPs.

A big problem in detecting nucleic acids by mass spectrometry consists in the negatively charged sugar-phosphate backbones⁸. Nucleic acids are multiply negatively charged polyanions that form salt adducts with cations used in reaction buffers, such as sodium and potassium, which significantly limits signal intensity and resolution. The tendency of cations to interfere with nucleic acids increases with oligomer length and is one of the main reasons for the limitations of MALDI-MS detection of large nucleic acids. Moreover, detergents that are commonly used in biological buffers are not compatible with MALDI matrix crystallisation. Therefore mass spectrometry analysis of nucleic acids requires stringent purification. Popular purification procedures comprise reversed-phase column purification, magnetic bead separation, ion exchange chromatography, gelfiltration, and ethanol precipitation^{6,8}. Most of these purification processes are cumbersome, partly expensive and time-consuming. The only MALDI-based methods for SNP genotyping, which circumvents stringent purification require a toxic DNA modification chemistry termed charge-tagging, which furthermore alters the hybridization properties (GOOD assays^{11,12} and related procedures using photocleavage¹³) . By charge-tagging nucleic acids can be modified in such a way that the sensitivity to salts and detergents is significantly reduced, allowing efficient MALDI detection without purification.

The development of microarrays was an important step in the miniaturised and high-parallel analysis of biomolecules³. Detection based on fluorescence is at present the method of choice to quickly screen the multiple (hundred-thousands) data points on microarrays. Classical microarray methods are however far too expensive, inflexible and eventually an overkill for diagnostics or candidate-gene studies. Alternative flexible and efficient diagnostic methods such as the well-established TaqMan^{™ 15} assay are costly and do presently not provide as high parallel detection of SNPs as arrays. More importantly, TaqMan^{™} detection is based on comparative (cluster) analysis of fluorescence signals. Fluorescence detection efficiently differentiates between positive and negative molecular events such as hybridisation or primer extension but it does not identify the analyte molecules.

In contrast to this, MALDI mass spectrometry analyses an intrinsic physical property of an analyte, the molecular weight. This allows very accurate diagnostic analysis.

Benters et al. (2002)¹⁸ described the application of DNA microarrays with PAMAM dendritic linker. Using these arrays, discrimination of SNPs can achieveded by varying hybridization conditions and subsequent measuring of fluorescence intensities. Purification is not performed on-chip, nor is there a suggestion to perform on-chip MALDI-MS.

Gobom et al. (2001)²⁷ described a MALDI sample preparation technique for peptide analysis. The peptide-specific hydrophobic MALDI matrix α-cyano-4-hydroxy cinnamic acid (CHCA) is used for purification purposes since it preferentially binds peptides. CHCA is provided on hydrophilic anchors coated on a metal target. Subsequently, solution containing peptide is delivered onto the solid CHCA. A water droplet is then applied to dilute salts and detergents of the peptide solution while the hydrophobic peptides stick on the hydrophobic matrix. The water droplet is removed after a while and the dried matrix-peptide sample can be efficiently detected by MALDI.

Sequenom developed the "MALDI on a chip" approach (see e.g. Little et al. (1997)¹⁶) for oligonucleotide analysis. In a first step an aqueous solution of the oligonucleotide-specific MALDI matrix material 3-hydroxypicolinic acid (3-HPA) is spotted in arrayed format, followed by spotting of purified oligonucleotides at the same locations. Purification is not addressed, let alone performed on-chip.

All prior art approaches to the mass spectrometric analysis of nucleic acids have in common that they require cumbersome purification or chemical modification of the nucleic acids prior to depositing the purified sample obtained thereby onto the MS target. In view of the limitations of the methods described in the prior art, the technical problem underlying the present invention was therefore the provision of facilitated means and methods for analyzing nucleic acids with mass spectrometry.

Accordingly, this invention relates to a method of analyzing nucleic acid with regard to composition and/or sequence comprising (a) bringing a sample comprising said nucleic acid into contact with positively charged and/or charge-neutral groups, said groups being attached to a solid support and under conditions allowing binding of said nucleic acid to said groups; (b) performing one or more washing steps; (c) depositing MALDI matrix material onto said solid support which has been contacted with said nucleic acid in step (a); and (d) performing MALDI-MS of said nucleic acid.

The term "nucleic acid" is understood to comprise oligo- and polydeoxyribonucleotides as well as oligo- and polyribonucleotides. In other words, all forms of DNA, e.g. genomic DNA and cDNA, and RNA, e.g. mRNA, tRNA, rRNA and genomic RNA (such as in case of RNA viruses) are embraced. The term "nucleic acid" furthermore comprises nucleic acid mimicking molecules known in the art which are capable of binding to positively charged or charge-neutral groups according to the invention. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA)³⁶. LNA is an RNA derivative in which the ribose ring is constrained by a methylene linkage between the 2'-oxygen and the 4'-carbon.

Furthermore embraced by the term "nucleic acid" according to the invention are derivatives of nucleic acids, i.e. molecules comprising one or more further groups or substituents different from those in naturally occurring nucleic acids. All groups or substituents known in the art and used for the synthesis of these molecules, such as protection groups, and/or for applications involving these molecules, such as labels and linkers, which are optionally cleavable, are envisaged.

The term "analyzing with regard to composition and/or sequence" according to the invention is to be read in conjunction with the envisaged read-out in step (c) of the method of the invention, said read-out being generated with mass spectrometry. Mass spectrometry provides data comprising the molecular mass, the mass-to-charge ratio (m/z) and/or the mass spectrum. The molecular mass is a molecular property and may differ from molecule to molecule of the same chemical species. This may be a result of the natural occurrence of more than one isotope of an atom type, which may apply to one or more of the atoms constituting the molecule(s) being analyzed. Alternatively or additionally, this may occur when more than one isotope of one or more constituent atoms was used for synthesis. In such cases the mass spectrum will exhibit more than one molecular peak, and intensities of the peaks will reflect the abundance of the different isotopes of the constituent atoms. The mass-to-charge ratio is the ratio m/z, wherein m is the molecular mass and z is the charge of the molecule when it is analyzed in the mass spectrometer. The m/z ratio is the property separation in the mass spectrometer relies on. The mass spectrum is a set of intensity and m/z data which may be displayed graphically in two dimensions as a plot intensity vs. m/z. Typically, peaks in a mass spectrum are very sharp (Dirac functions). Consequently, and as familiar to the skilled person, the molecular mass, the mass-to-charge ratio (m/z) and/or the mass spectrum are understood as being indicative of composition and/or sequence of the nucleic acid(s) being analyzed.

In other words, a sample comprising one or more distinct nucleic acids species will give rise to a mass spectrum with one or more peaks, each peak corresponding to one nucleic acid species, wherein, as discussed above and depending on the different isotopes present, one nucleic acid species (as such characterized by its sequence) may give rise to more than one molecular peak. Said mass spectrum with one or more peaks, each peak (or group of peaks in case more than one isotope of at least one constituent atoms is present) corresponding to a nucleic acid species, provides the desired information on the composition of the sample.

The determination of nucleic acid sequence by mass spectrometry can be performed, for example, as follows: Sequencing reactions, such as primer extension reaction and minisequencing reactions further detailed below, typically yield a series of products which differ in length by one nucleobase. Therefore, analysis of the reaction mixture with the method of the invention gives a mass spectrum with a series of distinct peaks, wherein the difference in mass between two subsequent peaks is the mass of the nucleobase added. By calculating the difference, the nature of the base is determined. Accordingly, the mass spectrum provides the desired sequence information.

The terms "positively charged groups" and "charge-neutral groups" relate to functional groups attached to a solid support which, upon establishing suitable conditions (see below), bind nucleic acid. Without being bound by a specific theory, binding of nucleic acid to positively charged groups involves charge-charge interactions, wherein the nucleic acid bears negative charge(s). Binding of nucleic acid to charge-neutral groups may involve charge-dipole and/or dipole-dipole interactions, the charge being located on the nucleic acid. The term "dipole" includes permanent, induced and fluctuating dipoles. Alternatively or additionally, said charge-neutral groups may be reactive groups which react to form a covalent bond to the nucleic acid.

Conditions allowing binding of nucleic acid to said groups include aqueous medium in a pH range where the nucleic acid molecule is overall negatively charged. Said pH range includes neutral pH (7.0), slightly acidic pH (including pH values of 5 and 6) and the entire range of alkaline pH values including pH = 8.

The term "solid support" relates to an object manufactured from solid materials such as glass, plastic or metal, which provides one or more surfaces amenable to the attachment of said groups. Said surface may be planar. Alternatively, the surface may exhibit a curvature. For example, the surface may be convex, such as a cylinder, sphere or microsphere. Said surface may be made of the same material as the bulk of the support. Alternatively, said surface may be coated with a distinct material. Preferred are coatings with materials facilitating the attachment of said groups.

The term "MALDI matrix material" is well known in the art and relates to a compound or a mixture of compounds capable of strongly absorbing energy at the wavelength of the laser used for desorption and capable of crystallizing with the analyte. Preferably, said MALDI matrix material does not or only to a small extent react chemically with the analyte, while being capable of proton transfer to the analyte.The term "MALDI-MS" is an abbreviation well known in the art and stands for matrix-assisted laser desorption/ionisation mass spectrometry. MALDI is method for the ionisation of molecules. Ionisation in turn is a prerequisite for performing mass spectrometry. MALDI is a method of ionisation, which, after its development in the 1980s turned out to be particularly suitable for peptides, nucleic acids and other large molecules including macromolecules such as polymers and biological macromolecules. MALDI involves the co-crystallization of an excess of matrix material (preferably 100- to 100000-fold molar excess) and the sample comprising the analyte. Short laser pulses (preferably 1 to 5ns) lead to excitation which, after relaxation in the crystal lattice leads to desorption of particles from the surface of the crystal. The matrix material, in addition to being capable of absorbing energy at the laser wavelength used, provides protection against fragmentation of large molecules. Mass spectrometry is characterized by low detection limit, large dynamic range, speed, accuracy and automation capability. Mass spectrometers are inherently multichannel detectors allowing the simultaneous detection of a plurality of analytes. Furthermore, there is no need for labelling. The MALDI process on the one side and the binding of nucleic acid to the abovementioned groups attached to the solid support on the other side are such that desorption takes place. In other words, said binding is reversible under desorption conditions.

In addition to the nucleic acid, further constituents of the sample may bind to said groups or said support. Washing is performed to ensure purity of the nucleic acid bound to the support. It is understood that the binding of nucleic acid to said groups on the one side and the conditions and stringency of the washing on the other side are such that binding of nucleic acid to said groups is maintained or substantially maintained, such that in step (d) of the method of the invention MALDI-MS of the nucleic acid bound to the solid support can be performed. Preferably said washing step(s) is/are effected with aqueous medium free of salts and buffer such as distilled water or bidistilled water. After washing, said support may be dried in a stream of dry gas such as dry nitrogen.

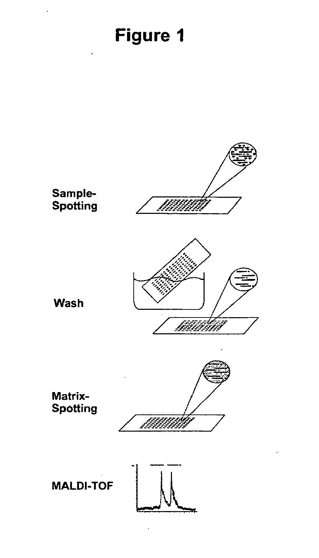

Figure 1 provides an illustration of the method of the invention.

The present inventors surprisingly recognized that solid supports carrying positively charged and/or charge-neutral groups, upon contacting with samples comprising nucleic acid(s) under conditions allowing binding of said nucleic, permit the purification of said nucleic acid samples to an extent sufficient for subsequent analysis by MALDI-MS. As a consequence, and as opposed to the prior art approaches, the MALDI matrix material is added after the DNA samples to be analyzed have been brought into contact with and have bound to the solid support. The method of the invention is also referred to herein as "on-target purification". This term indicates that all steps from purification of the crude sample to determination of the mass spectrum are performed on the same support or carrier, thereby providing convenient and efficient handling. The overall time-consumption as well as costs of purification and subsequent MALDI-MS analysis are significantly reduced as compared to the methods known in the art. Furthermore, on-target purification - as opposed to purification as known in the art - provides at least equal or increased sensitivity.

In a preferred embodiment of the method of the invention, said nucleic acid is obtained from a hybridization reaction, an oligonucleotide ligation reaction, an enzymatic digestion or a primer extension reaction and said analyzing comprises determining sequence, sequence variation and/or determining presence of an allele, haplotype or SNP.

The term "hybridization" as used herein refers to a pairing of a nucleic acid to a complementary strand of nucleic acid, which thereby form a hybrid. Said nucleic acids may be used as probes in Northern or Southern Blot analysis of RNA or DNA preparations, respectively, or can be used as oligonucleotide primers in PCR analysis dependent on their respective size. Preferably, said hybridizing polynucleotides comprise at least 5, more preferably at least 10, 12 or 15 nucleotides in length while a hybridizing polynucleotide of the present invention to be used as a probe preferably comprises at least 30, more preferably at least 50, 100 or 200, or most preferably at least 500 nucleotides in length.

It is well known in the art how to perform hybridization experiments with nucleic acid molecules, i.e. the person skilled in the art knows what hybridization conditions s/he has to use in accordance with the present invention. Such hybridization conditions are referred to in standard text books such as "Molecular Cloning A Laboratory Manual", Cold Spring Harbor Laboratory (1989) N.Y. or Higgins, S.J., Hames, D. "RNA Processing: A practical approach", Oxford University Press (1994), Vol. 1 and 2. "Stringent hybridization conditions" refers to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1 x SSC at about 65°C. Said conditions for hybridization are also known by a person skilled in the art as "highly stringent conditions for hybridization". Also contemplated are lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1 X SSPE, 0.1% SDS. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

In the primer extension reaction according to the invention the target sequence is annealed to a primer. Dideoxyribonucleotides (ddNTPs) and DNA polymerase are added to the mixture and the primer is extended by a single nucleotide. The single nucleotide added is dependent on the allele of the amplified DNA. Primer extension biochemistry has been coupled with a variety of detection schemes, comprising fluorescence, fluorescence polarization (FP), luminescence and mass spectrometry (MS). Said primer extension may be the extension of the primer by one nucleotide. This is preferred in applications where the first position immediately adjacent to the primer is a polymorphic position such as a SNP (see also references 23 to 26). Alternatively, 2 to 5, about 10 or about 20 nucleotides may be added to the primer in the course of said primer extension reaction. In these cases, the reaction is also referred to as minisequencing reaction. The nucleotides added to the primer in these cases may or may not comprise positions complementary to a polymorphic site of the template.

For oligonucleotide ligation (OLA), two specific oligonucleotides with a length of, for example, up to 30 nucleotides that anneal adjacent to each other on a DNA template are linked to each other by a DNA ligase in case of a complete complementarity between oligonucleotides and target sequences. The presence or absence of a ligated product identifies alleles of point mutations and SNPs.

Preferred enzymatic digestions are restriction enzyme digestion, flap endonuclease digestion and 5'-nuclease digestion.

MALDI mass spectrometry detection of SNPs by creating or deleting recognition sites for restriction enzymes, similar to gel-based restriction fragment length polymorphisms (RFLP) analysis is known in the art^{37,38}. Either naturally occurring restriction sites are used or nucleobase changes are incorporated in one of the amplification oligonucleotides to provide a recognition site with one of the alleles of the polymorphic position.

The Invader assay³¹ is based on the cleavage of specific nucleic acid structures by the 5'-nuclease domain of eubacterial Pol A DNA polymerases and structurally homologous FLAP endonucleases (FENs), which are involved in the DNA repair process^{32,33}. These enzymes, as well as genetically engineered thermostable versions thereof termed cleavases, specifically recognise and cleave an overlapping structure of two oligonucleotides that are hybridised adjacently to each other on a DNA template. The upstream oligonucleotide is termed invader and the downstream oligonucleotide probe. The 5'-end of the allele-specific probe contains an unpaired sequence called flap that is cleaved if the correct invasive structure is formed; in this way the invader displaces the probe. For the formation of the DNA triplex structure, probe and invader must overlap at least by one nucleotide, and the probe is cleaved 3' of the flap. Although a mismatch on the 3'-end of the invader oligonucleotide is tolerated, a mismatch between probe and template immediately upstream of the cleavage site precludes cleavage. An isothermal cleavage reaction leads to linear accumulation of product, in which uncleaved probes replace the cleaved ones. MALDI mass spectrometry was used as detection tool in connection with a so-called squared Invader format^{34,35}. In this procedure, the released overlapping sequence of the probe is used as an invader molecule in a secondary reaction, leading to an exponential amplification of products that can be detected with higher sensitivity.

In another 5'-nuclease assay (TaqMan) (Reference 15) a labelled probe oligonucleotide complementary to an internal sequence of the target DNA is added to a PCR. This oligonucleotide carries a fluorescence dye and a quencher. In case of plain hybridisation the probe oligonucleotide is degraded leading to separation of fluorescence dye and quencher, which promotes fluorescence light.

Any other procedure using exo- or endonucleases, DNAses or RNAses that leads to allele-specific products, which in turn can be detected by mass spectrometry is envisaged.

In a further preferred embodiment, said nucleic acid sample is a crude sample. Examples of crude samples are samples comprising, in addition to nucleic acids, buffers, salts, detergents and/or chemicals. As stated above, purification is a prerequisite for MALDI-MS since particularly salts and detergents are deleterious to matrix crystallization; furthermore salts form adducts with nucleic acids, which leads to decreased signal intensities and resolution in the mass spectrum. The recited washing step(s) ensure(s) that the bound nucleic acid is in purified or substantially purified form as compared to said sample. Additionally, a preferential binding of nucleic acids (as compared to the binding of detergents and/or salts) by the groups may occur such that a partial purification occurs as a result of said preferential binding.

In a further preferred embodiment, said positively charged groups are primary amino groups. In a further preferred embodiment, said charge-neutral groups are epoxy groups. Without being bound by a specific theory, it is envisaged that the epoxy ring opens and a covalent phosphodiester bond is formed with nucleic acid. After contact with acids such as 3-hydroxypicolinic acid (DNA matrix) and/or under desorption conditions, the bond would be cleaved and the previous epoxy ring would be converted into the corresponding 1,2-dihydroxy compound .

In a further preferred embodiment of the method of the invention, said groups are attached to said solid support by a three-dimensional structure such that an enhanced density of said groups is provided. The term "three-dimensional structure" according to the invention relates to any molecular structure which provides a third dimension to the - as such two-dimensional - surface of said solid support. More specifically, individual groups attached to said three-dimensional structure are located at different distances from the surface of the solid support, some of the groups closer to the surface, some of them more distant therefrom. This situation is to be distinguished from the use of linkers well known in the art. Linkers are typically molecules of the same length and as a consequence groups attached to the linkers are located at similar distance from the surface of the support. Since said three-dimensional structure provides for said groups being located within a range of distances from the surface of the support, it also provides for a density of said groups higher as compared to a situation where said groups are attached to the surface of the support either directly or by means of a linker. The person skilled in art is aware of such three-dimensional structures, which may be chosen from a broad range of chemical compounds. Particularly envisaged are polymers or polycondensates with a sponge-like, foam-like or tree-like structure. The molecules forming said three-dimensional structure may be attached to the surface of the solid support by coupling chemistry known in the art.

In a more preferred embodiment, said three-dimensional structure comprises or consists of (a) branched molecule(s), wherein said branched molecule carries a plurality of said groups.

In a yet more preferred embodiment, said branched molecule is a dendrimer, preferably a polyamidoamine (PAMAM) dendrimer. The term "dendrimer" is well known in the art and relates to polymeric chemical compounds having a tree-like structures with multiple branches. In the synthesis of dendrimers from monomers, monomers are used which exhibit a plurality of reactive groups, preferably three or more reactive groups. Upon repeated cycles of synthesis, dendrimers with increasing numbers of terminal groups are obtained, thereby providing a high density of the terminal groups. A particularly preferred dendrimer is PAMAM starburst dendrimer described in Benters et al. (2002)¹⁸.

The terms "polymer" and "polymeric" relate to both polymers in the narrow sense, i.e. molecules formed from a plurality of building blocks or one or more than one type (in the latter case said polymers are also referred to as co-polymers), wherein upon formation of the polymer from the building blocks no further molecule(s) such as water is formed, as well as to polycondensates, i.e. polymers according to the present invention, wherein upon formation of the polymer from its building blocks (a) further molecule(s) such as water is/are formed in addition to the polymer.

In a further preferred embodiment, said MALDI matrix material comprises or consists of 3-hydroxy picolinic acid (3-HPA), picolinic acid, 2,3,4-trihydroxyacetophenone, 2,4,6-trihydroxyacetophenone, 6-aza-2-thiothymine, α-cyano-4-hydroxy cinnamic acid methyl ester, or any combination thereof. These matrix materials are preferably to be used in conjunction with a laser wavelength in the ultraviolet range, preferably between about 200 nm and about 400 nm. Particulary preferred is a wavelength of 337 nm. Alternatively, and in a case where particularly mild desorption is needed, infrared lasers may be used, preferably with a wavelength between about 0.8 µm and about 400 µm. Particularly preferred is a wavelength of 2.94 µm. The use of infrared lasers entails the need for different matrix materials such as glycerol.

In a preferred embodiment, said solid support is selected from glass coated with gold, plastic coated with gold and metal coated with gold. In those embodiments of method of the invention, where polyamidoamines are to be coupled to supports coated with gold, the coupling may be effected with thiol chemistry.

In a further preferred embodiment of the method of the invention, said bringing into contact and/or said depositing is performed in parallel. Parallel handling preferably makes use of microtiter plates as containers for said nucleic acid samples. The arrayed arrangement of the wells of said microtiter plates is preferably preserved when samples are spotted onto the solid support of the invention. Microtiter plates preferably have 96, 384 or 1536 wells. Alternatively or additionally, nanowell plates may be used. Nanowell plates provide up to about 500 000 wells on a plate surface with commonly used dimensions.

In a further preferred embodiment, said bringing into contact and/or said depositing is performed by a robot.

In a further preferred embodiment of the method of the invention, a baking step is performed after step (a) and before step (b).

The baking step is preferably effected by placing the solid support into a thermostated chamber at temperatures elevated as compared to room temperature. The term "elevated temperature" includes temperatures in an interval between about 30°C and 150°C, including temperatures of 50°C, 60°C, 70°C, 80°C, 90°C and 100°C. The duration of the baking step is preferably between 10 seconds and 1 hour, including durations of 30 sec, 1 min, 2 min and 5 min.

In a further preferred embodiment, said nucleic acid brought into contact in step (a) is provided in a volume of about 100 nl, 10nl or 5nl or 1nl. Also preferred are embodiments, wherein said nucleic acid is provided in an amount of about 1, 0.5, 0.2 or 0.1 fmol. These embodiments are directed to a method of the invention wherein particularly small volumes and/or particularly small amounts of nucleic acid are handled. They lean themselves particularly to miniaturization, automation and high throughput. Parallelization and miniaturization permit reduction of reagent and DNA consumption.

The present invention also relates to a kit comprising (a) a solid support carrying positively charged and/or charge-neutral groups; (b) a vial containing MALDI matrix material; and (c) a manual with instructions for performing the method of the invention.

In a preferred embodiment of the kit of the invention, said positively charged groups are primary amino groups, said charge-neutral groups are epoxy groups and/or said MALDI matrix material comprises or consists of 3-hydroxy picolinic acid (3-HPA), picolinic acid, 2,3,4-trihydroxyacetophenone, 2,4,6-trihydroxyacetophenone, 6-aza-2-thiothymine, α-cyano-4-hydroxy cinnamic acid methyl ester, or any combination thereof.

The Figures show:
Figure 1: Exemplification of the procedure. Crude sample (SNP typing) reaction solution containing oligonucleotides is spotted onto an amino-coated, positively charged microscope slide surface and the droplets are let to dry. Then the slide is washed by dipping it several times in water. Afterwards MALDI matrix is spotted at the sample positions and the oligonucleotides are detected in a MALDI mass spectrometer.

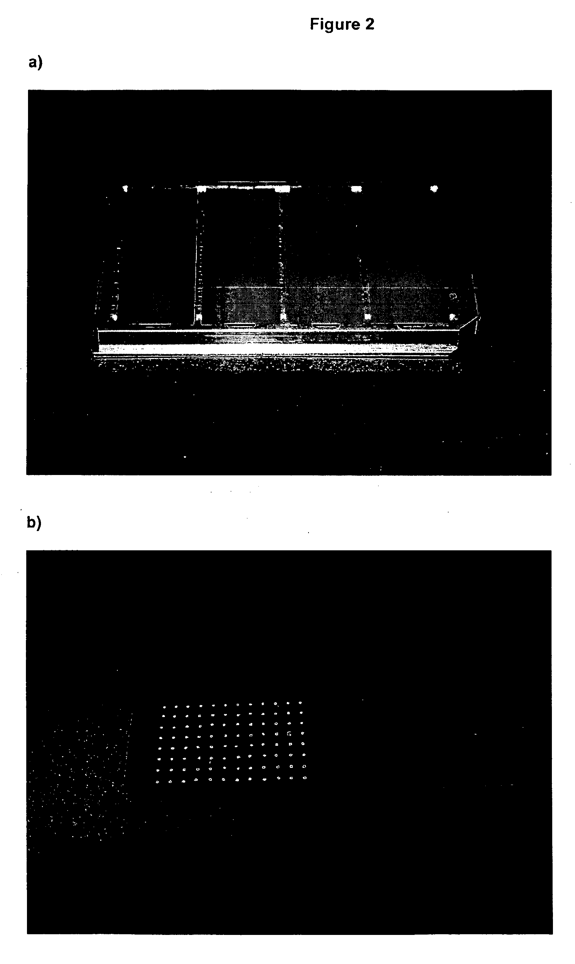

Figure 2: Gold microscope slides (modified with polyamidoamine (PAMAM) starburst dendrimers as mediator moieties and primary amino groups in the outer sphere) are clamped in home-made adapters as shown in a). The pale dots on photo b) are MALDI matrix spots disposed by the Flex array robot. For the slides in this figure ninety-six samples were spotted.

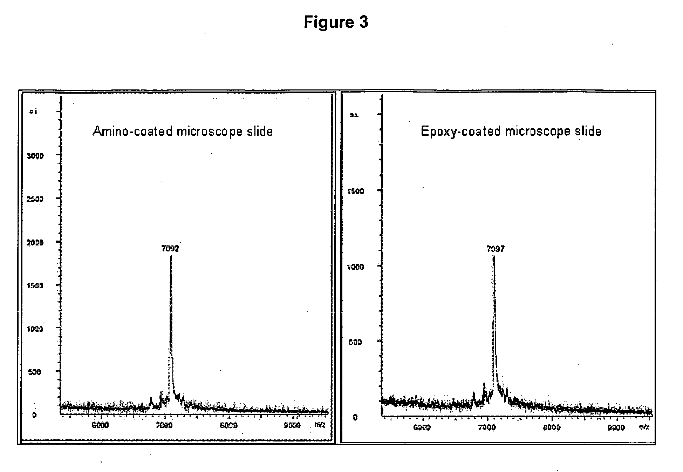

Figure 3: Two different coatings for the microscope slides were used. An oligonucleotide has been purified and detected by MALDI as described in the text. The left side mass spectrum shows a result using an amino-coated microscope slide, while the right hand mass spectrum shows a result using an epoxy-coated microscope slide. Typical mass differences result from preparation of HPA. Spectra were not smoothed or otherwise manipulated.

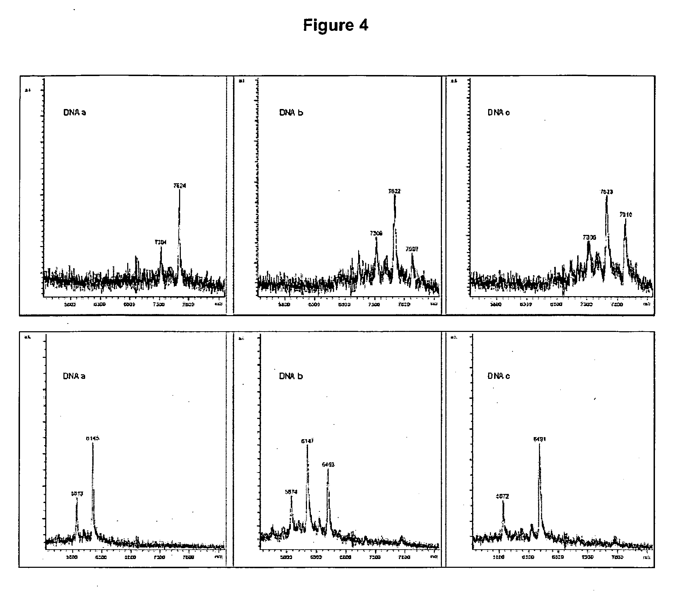

Figure 4: The upper mass spectra show products of SNP rs510769, the spectra on bottom show products of SNP rs607759. For SNP rs510769 primer TATGGCATTTCACATTCACATGTA (7300.98 Da) was used in the extension reaction. Respective products were TATGGCATTTCACATTCACATGTAG (7630.2 Da) and TATGGCATTTCACATTCACATGTAAT (7918.4 Da).

For SNP rs607759 primer AATTGAATGGCTCTAGGAC (5850.02 Da) was used in the extension reaction. Respective products were AATTGAATGGCTCTAGGACT (6154.22 Da) and AATTGAATGGCTCTAGGACTG (6483.44 Da). All masses are theoretical masses for positive ion mode.
For both SNPs three mass spectra are shown representing the three possible genotypes. Mass differences result from preparation of 3-HPA. Spectra were not smoothed or otherwise manipulated.

The following examples illustrate the invention but should not be construed as being limiting.

### Example 1

### On-target purification of oligonucleotides and MALDI detection

Materials and Methods described in this Example have also been used in the experiments described in the subsequent Example.

Unmodified oligonucleotides were obtained from MWG (Ebersberg, Germany). dNTPs and ddNTPs were obtaineded from Biolog (Bremen, Germany). Taq Polymerase was produced in-house (Roman Pawlik, Max-Planck Institute for Molecular Genetics, Berlin, Germany). TermiPol DNA polymerase was purchased from Solis Biodyne (Tartu, Estonia) and shrimp alkaline phosphatase was purchased from Amersham Buchler (Braunschweig, Germany). Chemical reagents were obtained from Aldrich (Steinheim, Germany). The MALDI matrix (3-hydroxypicolinic acid), and the GenoTools software are products from Bruker Daltonik (Bremen, Germany). The thermocycling procedures were carried out in a MJ PTC 200 Thermocycler obtained from Biozym (Hess. Oldendorf, Germany). Plasticware was purchased from Abgene (Hamburg, Germany) and Eppendorf (Hamburg, Germany). Gold microscope slides were obtained from Nunc (Wiesbaden, Germany). PAMAM dendrimer coating (amino coating and carboxyl-coating) was done by Chimera Biotec (Dortmund, Germany). The Flex array robot (Scienion, Berlin, Germany) was applied for spotting of reaction solution and MALDI matrix.

### Sample preparation:

We used an oligonucleotide (AATGTGATATTTTAAAGGGCCCT), diluted in a solution containing 0.33µl Taq DNA polymerase, 0.21µl 50mM MgCl2, 0.25µl SAP 1U/µl, 1.75µl TrisCl pH 8.0, 0.32µl TermiPol 5U/µl and 0.5µl oligonucleotide (10µM). We filled the mix up to a final volume of 6 µl using bidistilled water.

Preparation of MALDI targets: We used PAMAM dendrimer microscope slides with amino- or epoxy-coating. For the genotyping experiments we spotted 4 nl of the reaction solutions onto the modified microscope slides. The droplets are let to dry at room temperature (usually in about fifteen minutes). In case of epoxy-coated surfaces the slides were additionally incubated at 90°C (usually for about 15min). Subsequently, the slides were dipped several times in a tub containing bidistilled water and dried the slides under a stream of nitrogen. Thereafter 4nl of 120 mM 3-hydroxypicolionic acid in 15 mM ammonium citrate in deionised water were spotted onto the oligonucleotide samples.

Mass spectrometry analysis: Microscope slides were placed in an in-house fabricated adapter (see Figure 2) and spectra were recorded on a Biflex III time-of-flight mass spectrometer (Bruker Daltonik Bremen, Germany). This mass spectrometer is equipped with a Scout MTP^{™} ion source with delayed extraction. Spectra were recorded in positive ion linear time-of-flight mode. Typical acceleration potentials were 18 kV. For delayed extraction, the extraction delay was 200 ns. On average 50 laser shots per spectrum were accumulated when 200 nl matrix was applied, or 10 shots in case of 4 nl matrix.

### Example 2

### SNP genotyping using amino-modified microscope slides by MALDI mass spectrometry

SNP genotyping was performed using standard procedures consisting of PCR, shrimp alkaline digestion and primer extension.

PCR: DNA was prepared as described recently¹⁹. For the amplification of a piece of genomic DNA containing SNP rs510769 or rs607759 the following protocol was used: 1 µl of genomic DNA (10 ng), 5 pmol of the forward and 5 pmol of the reverse primer were mixed in 20 mM Trisbase, 16 mM (NH₄)₂SO₄, 25 mM KCl and 2 mM MgCl at pH 8.8 with 100 µM dNTPs and 10 U Taq DNA polymerase. The PCR was performed in a 10 µl volume. The reaction was denatured at 95°C for 4 min, then thermocycled for 15 s at 95°C, 30 s at 56°C and 60 s at 72°C, repeating the cycle 40 times.
Sequences of forward and reverse primers:
rs 607759 for: AAGCTCTAAAACATGGAAAGGAAA
rs 607759 rev: TCATGCAATGAAGGGGTCTTAT
rs 510769 for: TGTGATGGGTGCTCTAGACAAA
rs 510769 rev: GAAACCTGCAATACTTGCTGAA

Shrimp alkaline phosphatase digestion: 0.25 µl (1 U/µl) of shrimp alkaline phosphatase (SAP) and 1.75 µl 50 mM Tris base (pH 8.0) were added to 3 µl PCR solution and incubated for 70 min at 37°C. The SAP was denatured for 10 min at 90°C.

Primer Extension: 5 µM primer, 1.6 U TermiPol DNA polymerase, 0.5 mM ddNTP1, 0.7 mM ddNTP2, and 0.6 mM dTTP were added to the PCR in a final volume of 7 µl followed by 35 cycles of 15 s at 94°C, 30 s at 55°C and 15 s at 72°C.
Sequences for used primer extension primers and added dNTPs and ddNTPs:
PE_rs607759: AATTGAATGGCTCTAGGAC, ddCTP (=ddNTP1), ddGTP (=ddNTP2), dTTP
PE_rs510769: TATGGCATTTCACATTCACATGTA, ddGTP (=ddNTP1), ddTTP (=ddNTP2), dATP

Purification and sample preparation as well as MALDI detection were performed as described for example 1.

We used three DNAs (10, 14, and 27, internal numbering), which have different genotypes for SNP rs607759. We performed PCR , subsequent digestion of residual dNTPs by shrimp alkaline phosphatase digestion and finally primer extension reaction. We transferred 4 nl of the reaction solution using a the Flex array spotting robot onto PAMAM-modified gold microscope slides with mentioned amino functionalities and let the droplets dry. The spots were conservatively spotted at distances resulting in coverage of the upper half of the microscope slide. We dipped the slides twice in water and after drying of the slides we spotted 4 nl MALDI matrix using the same spotting robot. We clamped the slides in an adapter for microscope slides (Figure 2a) and analysed the samples in a conventional MALDI mass spectrometer.

To assess data quality we used GenoTools 1.0 software²¹: The settings of the software were similar to the default parameters of the parameter set termed "hard". The lower limit value was increased to 0.3. and the calibration tolerance was set to 15. We searched for signals in the appropriate mass ranges (5000-7000 m/z). Typical spectra are shown in Figure 4. The quality of these spectra was similar to those observed with larger volumes (200 nl) that were manually applied, however with significantly better reproducibility in the MALDI analysis due to more uniform matrix crystal formation.

### Further References

¹ Weeks, D.E. and Lathrop, G.M. (1995) Polygenic disease: methods for mapping complex disease traits. Trends Genet., 11, 513-519.
² Lander, E.S. and Schork, N.J. (1994) Genetic dissection of complex traits. Science, 265, 2037-2048.
³ Sauer, S. et al. Miniaturization in functional genomics and proteomics. (2005) Nat. Rev. Genet. 6, 465-476.
⁴ Pusch, W., Wurmbach, J.H., Thiele, H. and Kostrzewa, M. (2002) MALDI-TOF mass spectrometry-based SNP genotyping. Pharmacogenomics, 3, 537-548.
⁵ Karas, M. and Hillenkamp, F. (1988) Laser desorption ionization of proteins with molecular masses exceeding 10000 daltons. Anal. Chem., 60, 2299-2303.
⁶ Sauer, S. (2005) Typing of single nucleotide polymorphisms by MALDI mass spectrometry: principles and diagnostic applications. Clin. Chim. Acta, in press.
⁷ Sauer, S. and Gut, I.G. (2002) Genotyping single-nucleotide polymorphisms by matrix-assisted laser-desorption/ionization time-of-flight mass spectrometry. J. Chromatogr. B Analyt. Technol. Biomed. Life Sci., 782, 73-87.
⁸ Jurinke, C. et al. (2005) A single nucleotide polymorphism based approach for the identification and characterization of gene expression modulation using MassARRAY. Mutat, Res,, 573, 83-95.
⁹ Gut, I.G. (2004) DNA analysis by MALDI-TOF mass spectrometry. Hum. Mutat., 23, 437-441.
¹⁰ McCullough, R.M., Cantor, C.R. and Ding, C. (2005) High-throughput alternative splicing quantification by primer extension and matrix-assisted laser desorption/ionization time-of-flight mass spectrometry. Nucleic Acids Res 33, e99.
¹¹ Sauer, S. et al. (2000) A novel procedure for efficient genotyping of single nucleotide polymorphisms. Nucleic Acids Res,, 28, e13.
¹² Sauer, S. and Gut, I.G. (2003) Extension of the GOOD assay for genotyping single nucleotide polymorphisms by matrix-assisted laser desorption/ionization mass spectrometry. Rapid Commun. Mass Spectrom., 17, 1265-1272.
¹³ Sauer, S., Lehrach, H. and Reinhardt, R. (2003) MALDI mass spectrometry analysis of single nucleotide polymorphisms by photocleavage and charge-tagging. Nucleic Acids Res., 31, e63.
¹⁴ Matsuzaki, H. et al. (2004) Genotyping over 100,000 SNPs on a pair of oligonucleotide arrays. Nature Methods 1, 109-111.
¹⁵ Holland, P.M., Abramson, R.D., Watson, R. & Gelfand, D.H. (1991) Detection of specific polymerase chain reaction product by utilizing the 5'----3' exonuclease activity of Thermus aquaticus DNA polymerase. Proc. Natl. Acad. Sci. USA, 88, 7276-7280.
¹⁶ Little, D.P., et al. (1997) MALDI on a chip: analysis of low-to subfemtomole quantities of synthetic oligonucleotides and DNA diagnostic products dispensed by a piezoelectric pipette. Anal. Chem. 69, 4540-4546.
¹⁷ Tang, K. et al. Chip-based genotyping by mass spectrometry. Proc. Natl. Acad. Sci.. USA, 96, 10016-10020 (1999).
¹⁸ Benters, R., Niemeyer, C.M., Drutschmann, D., Blohm, D. and Wohrle, D. (2002) DNA microarrays with PAMAM dendritic linker systems. Nucleic Acids Res., 30, e10.
¹⁹ Burgtorf, C. et al. (2003) Clone-based systematic haplotyping (CSH): a procedure for physical haplotyping of whole genomes. Genome Res., 13, 2717-2724.
²⁰ Sauer, S. et al. (2004) Automated solid-phase extraction for purification of single nucleotide polymorphism genotyping products prior to matrix-assisted laser desorption/ionisation time-of-flight mass spectrometric analysis. J. Chromatogr. A, 1049, 9-16.
²¹ Pusch, W., Kraeuter, K.O., Froehlich, T., Stalgies, Y. and Kostrzewa, M. (2001) Genotools SNP manager: a new software for automated high-throughput MALDI-TOF mass spectrometry SNP genotyping. Biotechniques, 30, 210-215.
²² Gobom, J. et al. (2002) A calibration method that simplifies and improves accurate determination of peptide molecular masses by MALDI-TOF MS. Anal. Chem. 74, 3915-3923.
²³ Syvanen, A.C. (2001) Accessing genetic variation: genotyping single nucleotide polymorphisms. Nat. Rev. Genet. 2, 930-942.
²⁴ Little, D.P., Braun, A., Darnhofer-Demar, B. and Koster, H. (1997) Identification of apolipoprotein E polymorphisms using temperature cycled primer oligo base extension and mass spectrometry. Eur. J. Clin. Chem. Clin. Biochem. 35, 545-548.
²⁵ Haff, L.A. and Smirnov, I.P. (1997) Multiplex genotyping of PCR products with MassTag-labeled primers. Nucleic Acids Res. 25, 3749-3750.
²⁶ Herbon, N. et al. (2003) High-resolution SNP scan of chromosome 6p21 in pooled samples from patients with complex diseases. Genomics 81, 510-518.
²⁷ Gobom, J. et al. (2001) Alpha-cyano-4-hydroxycinnamic acid affinity sample preparation. A protocol for MALDI-MS peptide analysis in proteomics. Anal. Chem. 73, 434-438.
²⁸ Krebs, S., Medugorac, I., Seichter, D. and Forster, M. (2003) RNaseCut: a MALDI mass spectrometry-based method for SNP discovery. Nucleic Acids Res., 31, e37.
²⁹ Stanssens, P. et al. (2004) High-throughput MALDI-TOF discovery of genomic sequence polymorphisms. Genome Res., 14, 126-133.
³⁰ Schatz, P., Dietrich, D. and Schuster, M. (2004) Rapid analysis of CpG methylation patterns using RNase T1 cleavage and MALDI-TOF. Nucleic Acids Res., 32, e167.
³¹ Lyamichev V, Mast AL, Hall JG, Prudent JR, Kaiser MW, Takova T, Kwiatkowski RW, Sander TJ, de Arruda M, Arco DA, Neri BP, Brow MA. 1999. Polymorphism identification and quantitative detection of genomic DNA by invasive cleavage of oligonucleotide probes. Nat Biotechnol 17: 292-296.
³² Hosfield DJ, Frank G, Weng Y, Tainer JA, Shen B. 1998. Newly discovered archaebacterial flap endonucleases show a structure-specific mechanism for DNA substrate binding and catalysis resembling human flap endonuclease-1. J Biol Chem 273: 27154-27161.
³³ Kaiser MW, Lyamicheva N, Ma W, Miller C, Neri B, Fors L, Lyamichev VI. 1999. A comparison of eubacterial and archaeal structure-specific 5-exonucleases. J Biol Chem 274: 21387-21394.
³⁴ Griffin TJ, Hall JG, Prudent JR, Smith LM. 1999. Direct genetic analysis by matrix-assisted laser desorption/ionization mass spectrometry. Proc Natl Acad Sci USA 96: 6301-6306.
³⁵ Griffin TJ, Smith LM. 2000a. Genetic identification by mass spectrometric analysis of single-nucleotide polymorphisms: Ternary encoding of genotypes. Anal Chem 72: 3298-3302.
³⁶ Braasch DA and Corey DR. 2001. Locked nucleic acid (LNA); fine-tuning the recognition of DNA and RNA. Chemistry & Biology 8: 1-7.
³⁷ Liu Y-H, Bai J, Zhu Y, Liang X, Siemieniak D, Venta PJ, Lubman DM. 1995. Rapid screening of genetic polymorphisms using buccal cell DNA with detection by matrix-assisted laser desorption/ionization mass spectrometry. Rapid Commun Mass Spectrom 9: 735-743.
³⁸ Srinivasan JR, Liu YH, Venta PJ, Siemieniak D, Killeen AA, Zhu Y, Lubman DM. 1997. Matrix-assisted laser desorption/ionization time-of-flight mass spectrometry as a rapid screening method to detect mutations causing Tay-Sachs disease. Rapid Commun Mass Spectrom 11: 1144-1150.

## Claims

1. A method of analyzing nucleic acid with regard to composition and/or sequence comprising
(a) bringing a sample comprising said nucleic acid into contact with positively charged and/or charge-neutral groups, said groups being attached to a solid support and under conditions allowing binding of said nucleic acid to said groups;
(b) performing one or more washing steps;
(c) depositing MALDI matrix material onto said solid support which has been contacted with said nucleic acid in step (a); and
(d) performing MALDI-MS of said nucleic acid.

2. The method of claim 1, wherein said nucleic acid is obtained from a hybridization reaction, an oligonucleotide ligation reaction, an enzymatic digestion or a primer extension reaction and said analyzing comprises determining sequence, sequence variation and/or determining presence of an allele, haplotype or SNP.

3. The method of claim 1 or 2, wherein said nucleic acid sample is a crude sample.

4. The method of any one of claims 1 to 3, wherein said positively charged groups are primary amino groups.

5. The method of any one of claims 1 to 4, wherein said charge-neutral groups are epoxy groups.

6. The method of any one of claims 1 to 5, wherein said groups are attached to said solid support by a three-dimensional structure such that an enhanced density of said groups is provided.

7. The method of claim 6, wherein said three-dimensional structure comprises or consists of (a) branched molecule(s), wherein said branched molecule carries a plurality of said groups.

8. The method of claim 7, wherein said branched molecule is a dendrimer, preferably a PAMAM dendrimer.

9. The method of any one of claims 1 to 8, wherein said MALDI matrix material comprises or consists of 3-hydroxy picolinic acid (3-HPA), picolinic acid, 2,3,4-trihydroxyacetophenone, 2,4,6-trihydroxyacetophenone, 6-aza-2-thiothymine, α-cyano-4-hydroxy cinnamic acid methyl ester, or any combination thereof.

10. The method of any one of claims 1 to 9, wherein said solid support is selected from glass coated with gold, plastic coated with gold and metal coated with gold.

11. The method of any one of claims 1 to 10, wherein said bringing into contact and/or said depositing is performed in parallel.

12. The method of any one of claims 1 to 11, wherein said bringing into contact and/or said depositing is performed by a robot.

13. The method of any one of claims 1 to 12, wherein a baking step is performed after step (a) and before step (b).

14. The method of any one of claims 1 to 13, wherein said nucleic acid brought into contact in step (a) is provided in a volume of about 100 nl, 10nl or 5nl or 1nl.

15. The method of any one of claims 1 to 14, wherein said nucleic acid is provided in an amount of about 1, 0.5, 0.2 or 0.1 fmol.

16. A kit comprising
(a) a solid support carrying positively charged and/or charge-neutral groups;
(b) a vial containing MALDI matrix material; and
(c) a manual with instructions for performing the method of any one of claims 1 to 15.

17. Kit according to claim 16, wherein said positively charged groups are primary amino groups, said charge-neutral groups are epoxy groups and/or said MALDI matrix material comprises or consists of 3-hydroxy picolinic acid (3-HPA), picolinic acid, 2,3,4-trihydroxyacetophenone, 2,4,6-trihydroxyacetophenone, 6-aza-2-thiothymine, α-cyano-4-hydroxy cinnamic acid methyl ester, or any combination thereof.
